# EUROPEAN PATENT APPLICATION

(11) **EP 4 342 380 A1**
(43) Date of publication of application: **27.03.2024**
(21) Application number: 22197507.1
(22) Date of filing: 23.09.2022
(51) Int. Cl.: A61B 5/369, A42B 1/045, A61B 5/00

(54) **CAP DEVICE FOR USE IN TAKING MEASUREMENT DATA FROM A HEAD OF A PERSON**

(71) Applicant: NIRx Medizintechnik GmbH, 13355 Berlin (DE)
(72) Inventor: BRITZ, Patrick, 10115 Berlin (DE); VON LÜHMANN, Alexander, 10119 Berlin (DE); NANDORI, Antonia, 6003 Luzern (CH); SOUNDARARAJAN, Jaghnivasan, 12247 Berlin (DE)
(74) Representative: Maikowski & Ninnemann Patentanwälte Partnerschaft mbB

(57) **Abstract**

The invention relates to a cap device (50) for use in taking of measurement data, in particular electrical or optical neuroimaging data from a head (H) of a person and / or for use in stimulating the neural functions of a person, with a chin belt device (20) comprising positioning means (2, 3, 4, 7, 9, 10, 14, 15, 30) for defining chin belt device's (20) position relative to the head (H) and / or the face of the person wearing the cap device (50), the positioning means (2, 3, 4, 7, 9, 10, 14, 15, 30) configured to define - when fastened - a force vector (F) in a median plane (M) of the person's body and at an angle between 10 and 45° measured against a plane (P) perpendicular to the median plane (M), approximately crossing the neck of the person in a standing position. The invention also relates to a cap device (50) for use in taking of measurement data, in particular electrical or optical neuroimaging data from a head (H) of a person and / or for use in stimulating the neural functions of a person,, with the cap device (50) comprising connections means (60) for a plurality of sensors (70), in particular optodes wherein, at least a part of the connection means (60) are located in a pattern comprising triangles (61) or hexagons (62), in particular regular triangles (61) or regular hexagons (62).

## Description

The invention relates to a cap device with the features of claim 1 and / or claim 2.

For research and /or diagnostic purposes, it can be necessary to take noninvasive measurements from the surface of a person's head using sensors, such as electrodes or optodes. Electrodes are, e.g., used for taking data for an electroencephalogram. In optical neuroimaging techniques, optodes are used to emit electromagnetic waves into the head of a person and sense how these signals are absorbed and backscattered by the components of the tissue.

For this, a plurality of sensors need to be positioned at and / or on the head of the person. This is generally achieved by fastening a plurality of sensors on and / or in a cap which is worn by the person. The sensors are e.g. in contact with the scalp of the person so that data can be collected from the sensors. The data and / or the signals are transmitted e.g. via cables to a processing device, such as a computer. The processing device then produces time traces or images using data cleaning and reconstruction methods.

The proper fastening of the sensors to the head is in particular important for the quality and reproducibility of the measurements and / or for brain stimulation, as precise co-localization of sensors / stimulators and individual subject anatomy is often crucial. This task is complicated by the fact that in humans the shape of the head *per se* is complex and the head has a high variability in size and shape. Furthermore, as the measurements are not only performed at rest, the positioning of the sensors should be invariant to movements or robust against movements of the person.

One domain of particular concern is high-density (HD) fNIRS - a method that employs high-density arrays with short, medium, and long channels. Overlapping measurements with multiple source-detector distances can improve spatial resolution, depth, and lateral specificity, and can enable reconstruction of depth-resolved functional cerebral activation (for instance by Diffuse Optical Tomography) (see e.g. Boas, D.A., Brooks, D.H., Miller, E.L., DiMarzio, C.A., Kilmer, M., Gaudette, R.J. and Zhang, Q., 2001. "Imaging the body with diffuse optical tomography". IEEE signal processing magazine, 18(6), pp.57-75; Bluestone, A.Y., Abdoulaev, G., Schmitz, C.H., Barbour, R.L. and Hielscher, A.H., 2001. "Three-dimensional optical tomography of hemodynamics in the human head". Optics express, 9(6), pp.272-286; Barbour, Randall L., et al. "Optical tomographic imaging of dynamic features of dense-scattering media." JOSA A 18.12 (2001): 3018-3036.

Therefore, caps providing secure and precise positioning of sensors and / or stimulators relative to the head are required.

The issue is addressed by a cap device with the features of claim 1 and / or claim 2.

This cap device is configured and designed for a use in taking measurement data, in particular electrical or optical neuroimaging data (e.g. electroencephalogram, EEG or functional Near-Infrared Spectroscopy, fNIRS) from a head of a person and / or applying brain stimulation signals, in particular electrical or magnetic signals (e.g., transcranial Direct Current Stimulation, tDCS, or low intensity focused ultrasound, LIFUS).

The fit of the cap device to the head is made with a chin belt device comprising positioning means (e.g. straps or belts) for defining chin belt device's position relative to the head and / or the face of the person wearing the cap device. The chin belt device works together with the person's chin as an anchor point for positioning the cap device properly relative to the head of the person. The positioning means are configured to define - when fastened - a force vector in a median plane of the person's body and at an angle between 10 and 45° measured against a plane perpendicular to the median plane, approximately crossing the neck of the person in a standing position. With the positioning means a defined fastening force is applied to the cap and - as a reaction force - it is applied on the chin. With this force arrangement, the cap device can be positioned properly even if the person is moving during the measurements.

The issue is also addressed alternatively or in addition by a cap device for use in applying stimulation and / or taking of measurement data, in particular electrical, magnetical, or optical neuroimaging data from a head of a person, with the cap device comprising connections means or a plurality of sensors, in particular optodes wherein, at least a part of the connection means is located in a pattern comprising triangles or hexagons, in particular regular triangles or regular hexagons. The arrangement of the sensors in the hexagonal or triangular pattern assures defined and homogeneously distributed relative distances between the sensors, even if the material of the cap device is stretched.

Three aspects are in particular relevant to obtain enhanced imaging quality:
1) A high dynamic range of the instrument and
2) excellent sensitivity are needed to measure good signals across a range of distances between optodes and
3) the arrangement of / distances between optodes needs to be precisely known and correctly set up to reduce errors in the inverse problem of the tomographic reconstruction and to make optimal use of 2) and 3) dynamic range and sensitivity.

The described patterns and embodiments of the cap device optimizes the above given items 1-3 while also maximizing the number of different overlapping channel distances that are feasible, and ensuring precise co-registration using flexible/stretchable textiles.

In the case of neural stimulation, the same applies as well, as the stimulation has to be performed in a defined location pattern. The signals of the stimulators - as mentioned above e.g. magnetic or electric - have to be applied in defined locations.

In one embodiment, the chin belt device with the positioning means is fastenable on the chin or under the chin of the person. The chin belt device can be connected together with the positioning means so that they can be applied together to the chin of the person. In one embodiment, the chin belt device can be one piece with the positioning means.

For an easier handling, in particular during donning of the cap device and during removing, the chin belt device can be configured removable from the cap device. With this, one would e.g. don the cap device first, then attach the chin belt device to it and then fasten the cap device together with the positioning means.

In one embodiment, the cap device comprises positioning means with a crossing strap device. The crossing strap device comprises a first crossing strap that is attachable to the cap device towards the neck portion of the cap device, the first crossing strap being in use essentially parallel to the plane perpendicular to the neck of the person in a standing position, and an upper strap attachable to the side portion of the cap device, the crossing straps crossing over in attaching to the chin belt device to tighten the fit on the face of the person. The crossing strap device allows for a specific force distribution around the head using the person's chin as some anchor point. With the first crossing strap (lower crossing strap), a force can be applied towards the neck portion and the portion under the ears of the person. With the second crossing strap (upper crossing strap), a force can be applied towards the side portions of the cap device. By exerting a force in the direction mentioned above, the chin and the crossing straps split the pull vector into two components, working at the same time on the two different portions of the cap device.

For a good fit of the cap device, a face string for fastening the cap device around the face of the person is provided in one embodiment. In particular, the face string is operable with one hand, so that a person can adjust the secure fit efficiently.

In one embodiment, the face string is configured so that there is no dangling part. This is achieved by fixing the face string to a part of the cap device. The string threads secured in a tubular sheath favoring localized tension on the forehead area and reducing slippage of the cap device. An increase/decrease in stress in the face string is achieved by pulling force through a pressure based interlocking system releasing/securing excess cord material from the sheath. The end of this tubular cord is sewed back in the cap making it easy to operate and avoid dangling.

To collect measurement data, the sensor on the cap device regularly is connected with cables to a processing device. The same is true for the connection of stimulators. One embodiment comprises at least one cable guiding means for cables attachable to the sensors and / or stimulators at and / or on the cap device. The at least one cable guiding means is positioned on the neck portion of the cap device. The cable guiding means can e.g. comprise at least one Velcro fastener for fastening cables at the neck portion of the cap device. Additionally or alternatively, an embodiment can comprise at least one cable guide element, in particular at least one cable tree at the neck portion of the cap device. With the cable guiding means the connection between the sensors and the processing means is secured, as the movement of the cables relative to the cap device is reduced which is particularly important if measurements are taken from moving persons.

In one embodiment, the cap device comprises an elastic textile material at and / or in the top and the side portions of the cap device with an even stretch capability at least in two perpendicular directions. One example for such a material is Powernet as a nylon based knitted material in the form of mesh structure that retains its shape and offers surface breathability.

As the cap device is seated on head and fastened to the head, one embodiment comprises a neck stiffening device, in particular comprising a neoprene lining, at and / or in the neck portion and / or the lower part of the side portion of the cap device. This stiffening device provides a better fit and makes the wearing more comfortable. In particular it is possible, to use the stiffening device also as a base for cable guiding means.

In one embodiment, all parts of the cap device are manufactured from plastic, textiles and / or non-magnetic material.

Some embodiments are shown in an exemplary way in the following figures.
- Fig. 1A-C: show an embodiment of a cap device in a right view, a frontal view and a left view;
- Fig. 2A-C: show an embodiment of a cap device in a right view, a frontal view and a left view with details related to the positioning means;
- Fig. 2D: shows a detail of a face string;
- Fig. 2E: shows a detail of a closing buckle;
- Fig. 2F: shows a cord stopper;
- Fig. 2G: shows a ladder buckle;
- Fig. 2H: shows a detail of the attachment of the closing buckle;
- Fig. 2I: shows a detail of the attachment of the chin cup;
- Fig. 3: shows the overall shape of an embodiment of a crossing strap device;
- Fig. 4: shows the attachment of the crossing strap device to one side of the cap device;
- Fig. 5: shows an embodiment of a neck stiffening device with cable guide elements;
- Fig. 6: shows an embodiment of a cap device with a cable tree as a cable guide element;
- Fig. 7A-C: show an embodiment of a cap device in a right view, a frontal view and a left view;
- Fig. 8A: shows a medial sectional view of a head with defining positions for connections means for sensors (e.g. an EEG 10-20 system);
- Fig. 8B: shows a top view of a head with defining positions for connection means for sensors;
- Fig. 9: shows a part of a cap device with a pattern for connection means and a centering position around the vertex of the head;
- Fig. 10: shows a part of a cap device with high density sensor arrangement.
- Fig. 11: shows a part of a cap device with a normal density sensor arrangement;
- Fig. 12: shows the connector means pattern for the cap device top portion;
- Fig. 13: shows the connector means pattern for a cap device side portion;
- Fig. 14: shows a detail view of a part of a cap device with connection means for sensors and optodes as sensors on a hexagonal pattern;
- Fig. 15: show she an embodiment of a crossing strap device.

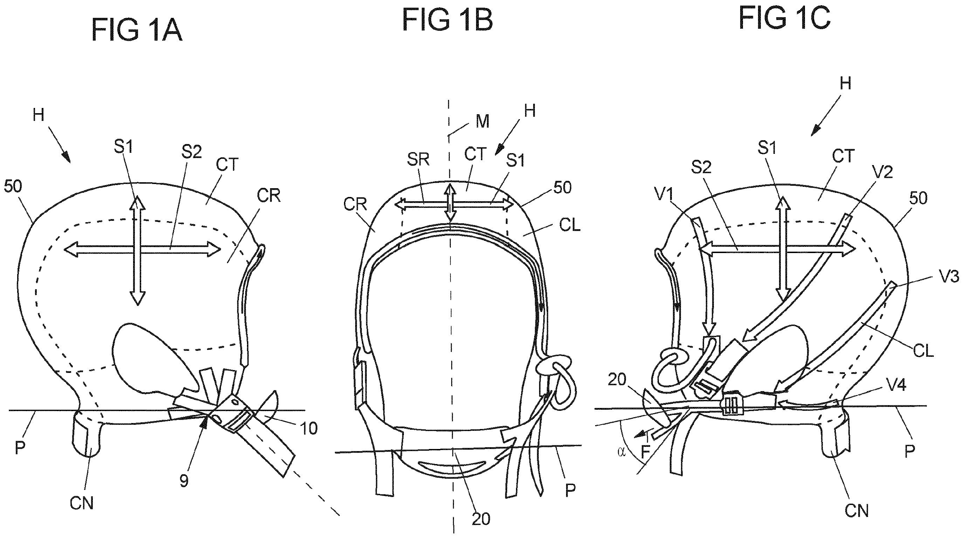

Fig. 1A-C show a cap device 50 worn on a head H of the person in three views. Fig. 1A is a view on the right hand side of the head H, Fig. 1B is a frontal view of the head H and Fig. 1C is a view on the left hand side of the head H.

In Fig. 1B the median plane M (symbolized by a line) of the head H is indicated, separating the head H into a left and right half.

In Fig. 1A to 1C also a horizontal plane P (again symbolized by a line) is shown. The horizontal plane P is perpendicular to the median plane M and crosses approximately the neck of the person in a standing position. The planes M, P will be used to define a force F to fasten the cap device 50 to the head H of the person.

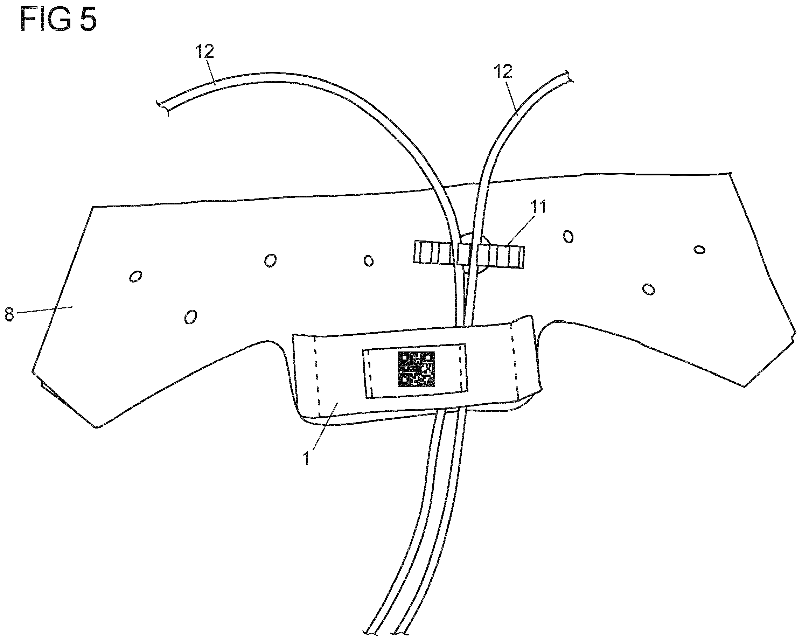

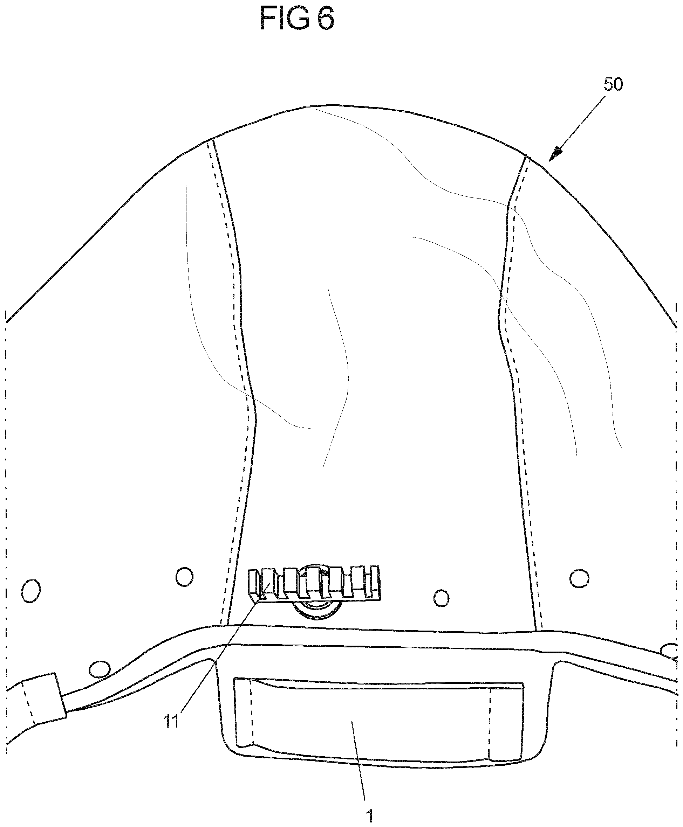

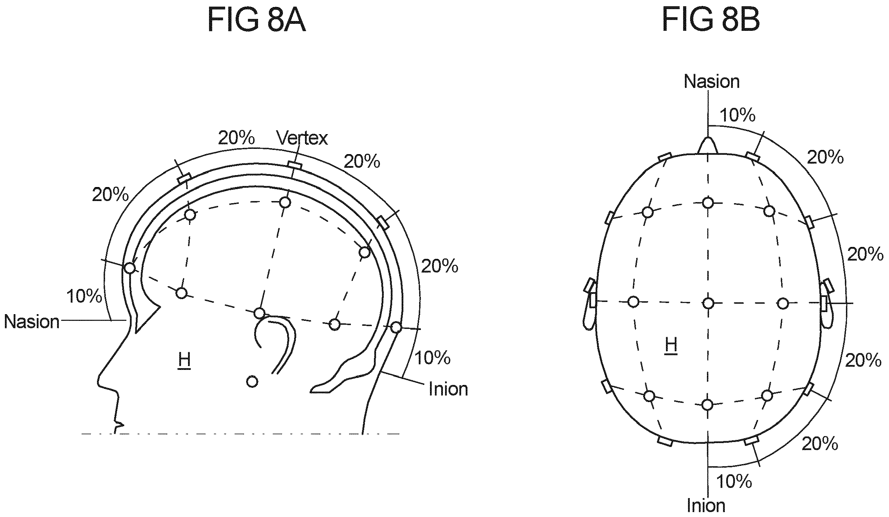

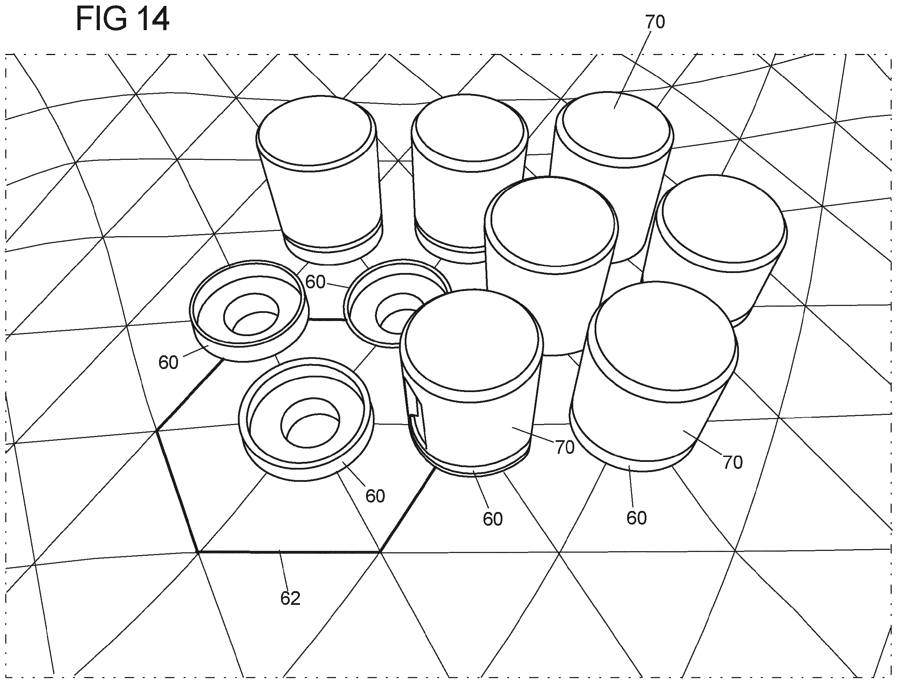

The cap device 50 is intended to carry sensors 70 such as electrodes or optodes for taking measurements from the head H (see Fig. 8A, 8B, 14). For reasons of simplicity the sensors themselves are not shown here. Embodiments using cap devices described in Fig. 1 to 7 and 15 with exact placement structures of the sensors 70 are described in connection with Fig. 8 to 14. The base material of the cap device 50 material is mostly a flexible, stretchable textile material (e.g. Powernet) to which certain additions are made (see e.g. Fig. 5 and 6).

The cap device 50 comprises several portions CL, CR, CN, CT which in the embodiment shown are separated by seams (dashed lines). The cap device top portion CT extends from the eye brow region, across the cranium to the neck of the person. The neck of the person itself is covered by the cap device neck portion CN. The sides of the head H are covered by the cap device left hand side CL and the cap device right hand side CR.

In Fig. 1A to 1C arrows S1, S2 perpendicular to each other in the top and side portions CT, CL, CR of the cap device 50 indicate an even stretch property of the textile material - such as Powernet - used in those parts of the cap device 50. This means that the material of the cap device 50 stretches at least evenly in those two different stretch directions S1, S2. Materials such as Powernet also stretch evenly in a direction under 45° to the indicated stretch directions S1, S2. Using an even-stretch material ensures that
a) the cap device 50 has a secure fit to head H of different shapes,
b) keeps the cap device 50 in place, in particular in the case of movements of the person, and
c) distances between sensors are defined across the cap device. In particular the sensors can be evenly distributed over the head H of the person. Distances are e.g. defined relative between the sensors 70 and relative to the head H. This makes sure that measurement results - in particular with different head sizes - are reproducible. The same holds true for the use of stimulators applied to a head H of a person. In the following the embodiments are described in the in the context of sensors 70. An analogue arrangement would also be used in the case of stimulators in those positions. It is in principle possible to use sensors 70 and stimulators in the same cap device 50 concurrently or at different times.

This secure fit is enabled by straps and parts 2, 3, 4, 7, 9, 10, 14, 15, 30 which will be described as parts of a positioning means further below.

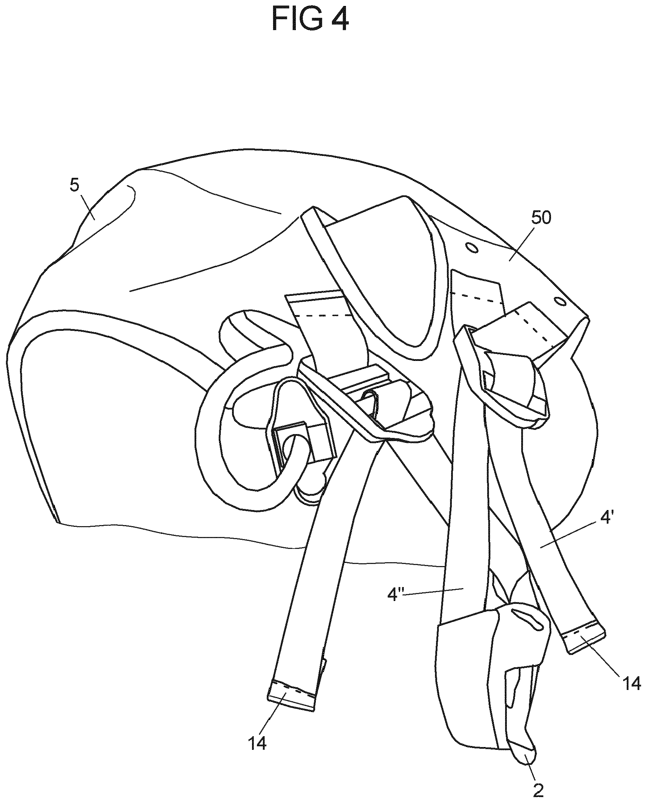

In Fig. 1C arrows V1, V2, V3, V4 indicate approximately regions of the left-hand side of the cap device 50 where with positioning means, i.e. here straps 3, 4, 4', 4", forces (see e.g. Fig. 4) can be applied.

Some force is applied to the frontal pull region V1 from where the textile material is pulled essentially downwards within the cap device left portion CL along the sides of the face to the chin region.

There is also a force applied to the median pull region V2, V3 which extends from the rear head diagonally across the cap device left portion CL to the chin region of the cap device 50.

And there is a lower pull region V4 which extends from the neck region CN towards the chin region of the cap device 50.

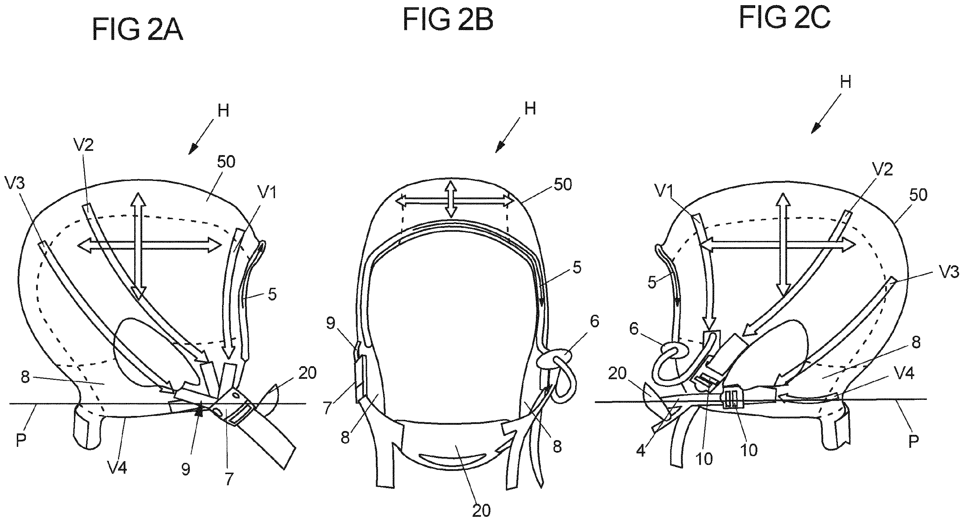

The same force pattern applies essentially to the right hand side of the head H, as is shown in connection with Fig. 2A.

As will be shown below, there are dedicated straps pulling on those three regions V1, V2, V3, V4 of the cap device 50, the forces converging on the chin region of the head H. It should be noted that the features described here, are mostly shown in different views in Fig. 2A to 3.

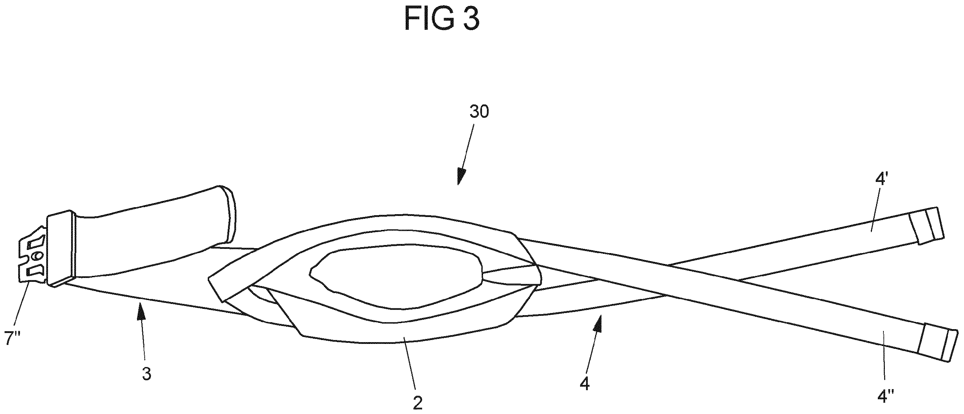

The positioning means, i.e. here the straps 3, 4, 9, define a chin belt device 20 (see e.g., Fig. 3, and also 15) position relative to the head H and / or the face of the person wearing the cap device 50. The chin belt device 20 comprises a cup-like part fitting onto the chin of the person.

The defined positioning is achieved by the positioning means 2, 3, 4, 7, 9, 10, 14, 15, 30 configured to define a force vector F by pull-forces at the straps 3, 4, 9, 14, 15 leading to a tightening of the stretchable material comprising the pull regions V1, V2, V3, V4. The resulting force vector F lies essentially in the median plane M of the person's body, i.e. it is vertically centered. The force vector F, furthermore, is at an angle α between 10 and 45° measured against the plane P perpendicular to the median plane M (see Fig. 1C). This means that the force vector F pulls essentially in the direction of the tip of the chin, i.e. there is a resulting force from the chin belt device 20 onto the chin. The general direction of the force vector F could also be described as forward and downward as seen from the person wearing the cap device 50.

The definition of the force vector F alone or in combination with an arrangement of the sensors 70 (see e.g. Fig. 8 to 13), e.g. with an even stretch material and an homogeneous distancing of the sensors 70 thus allows for precise co-registration and improved neuroimaging (image reconstruction) performance.

It should be noted that the shape and size of the cap device portions CL, CN, CL, CT can be designed differently in other embodiments.

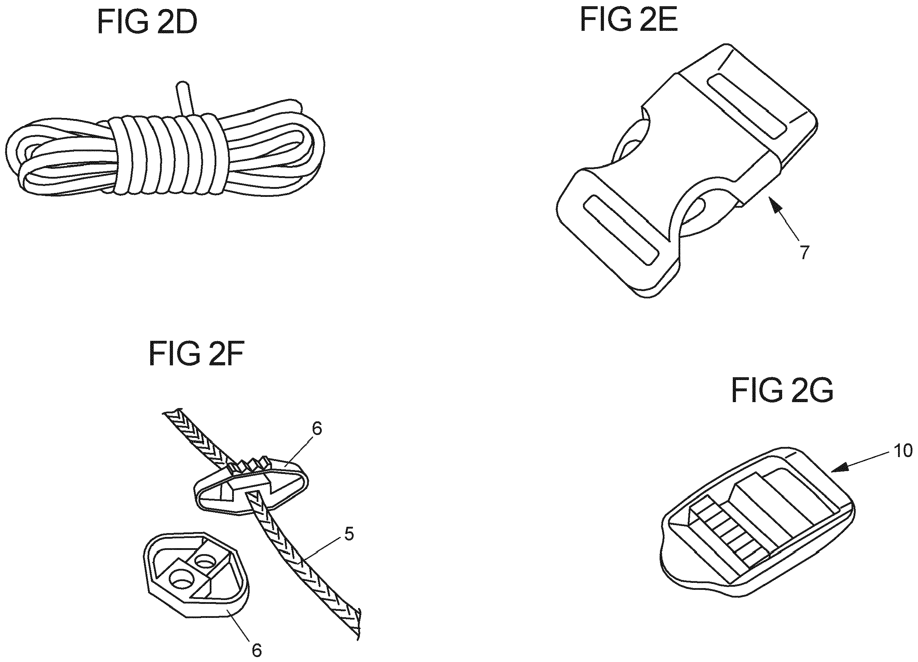

In Fig. 2A to 2C the same situation of a head H with a cap device 50 is shown as in Fig. 1A to 1C. Therefore, reference can be made to the above description. Details of accessories for the cap device 50 are shown in detail in Fig. 2D to 2G.

As already mentioned, the cap device 50 comprises a chin device 20 with a chin cup 2 form fitting with the chin of the person. The chin cup 2 together with straps 3, 4 comprises a part of the positioning means that presses the chin cup 2 against the chin when the straps 3, 4 are fastened. The chin cup 2 does not have to be a closed shape like a cup, but it can also be formed from straps - as will be shown below e.g. in Fig. 3 - forming something like a cup for the chin.

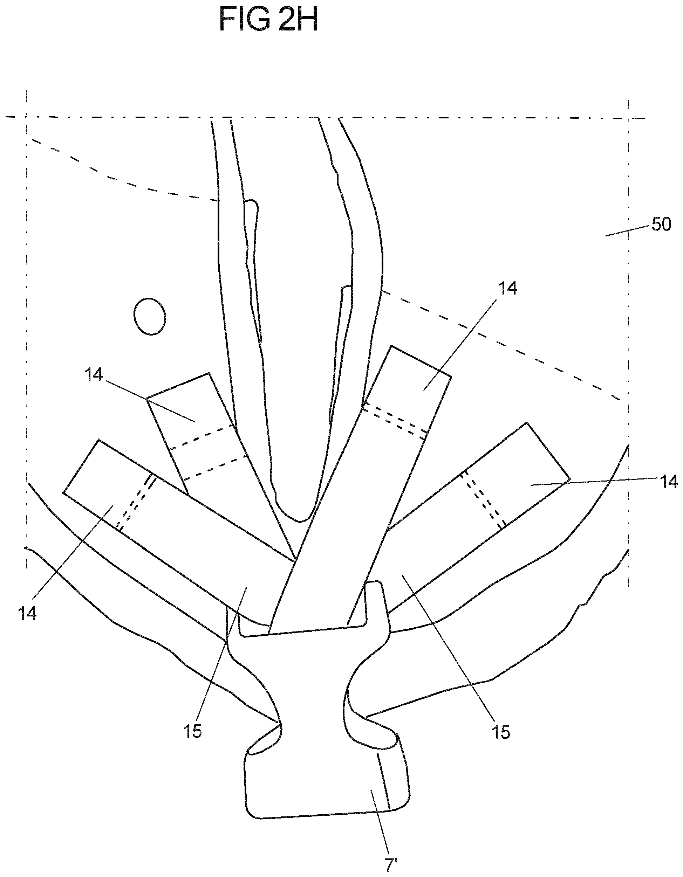

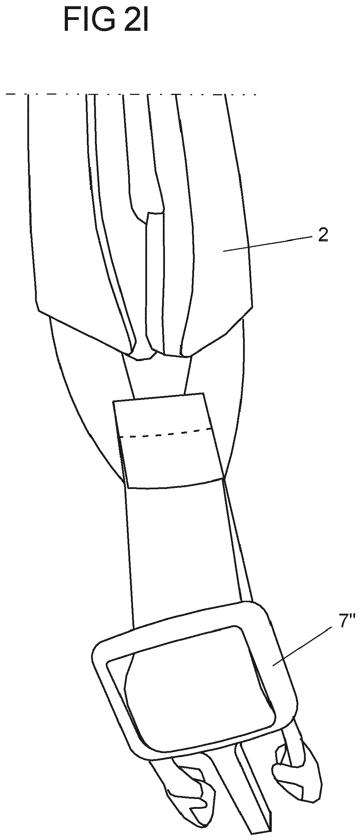

In the embodiment shown in Fig. 2A to 2C, the positioning means further comprises a closing buckle 7 (see Fig. 2E) which is on a first end (female part 7') attached to four strap ends 14 attached to the cap device 50. The four strap ends 14 are the ends of two short straps 15 forming two loops by being fold over, as shown in Fig. 2H. The loops are attached to the female part 7' of the closing buckle 7. The male part 7" of the closing buckle 7 is shown in Fig. 2I and Fig. 3.

By forming the loops, the strap ends 14 form crossing straps 9, i.e., the strap ends 14 fan out on the side of the cap device 50 to allow a defined and spread application of a pull force applied to the cap device 50, as e.g. seen in Fig. 1A or 2A.

Through the strap ends 14 a pull on the four pull regions V1, V2, V3, V4 of the right-hand side of the cap device 50 can be applied, as indicated by the arrows in Fig. 2A. The pull on the right-hand side of the cap device 50 works analogously as described for the left-hand side (see Fig. 1C), only the strap ends 14 designed slightly different to allow for a tightening mechanism. Together with the pulling on the cap portions on the right hand side (see Fig. 1C) an even force or stress distribution in the cap device 50 is achieved.

When donning the cap device 50, the closing buckle 7 is closed with its first end, i.e. the female part 7' on the right hand side of the cap device 50.

The second end of the closing buckle 7, the male part 7" is connected with the chin cup 2 through a strap best seen in Fig. 21.

The chin cup 2 is part of a crossing strap device 30 shown in Fig. 3. This comprises a strap 3 for the right hand side of the cap device 50, here shown with the male part 7" of the closing buckle 7 on one side of the chin cup.

On the opposite side, the chin cup 2 has two straps 4', 4" attached for the left hand side of the cap device 50. Those two straps 4', 4" are crossed (see Fig. 4) so that they can be attached in this crossed position to the left hand side of the cap device 50.

These crossed two straps 4', 4" (i.e. the crossing straps) are connected to the four pull regions V1, V2, V3, V4 on the left hand side of the head H, as described above. There are loose strap ends which can be pulled essentially downwards to fasten the cap device 50 with the positioning means 2, 3, 4, 7, 9, 10, 14, 15, 30 to the head H. Ladder buckles 10 (see Fig. 2G) as a part of the positioning means make sure that the straps remain tightened.

In the following and in connection with Fig. 4, the attachment of the crossing strap device 30 to the left hand side of the cap device 50 is described in more detail. This crossing strap device 30 is essentially the chin device 20 shown e.g. in Fig. 1A.

In Fig. 4, the part of the cap device 50 is shown to which the crossing strap device 30 is attached to.

Dangling downwards, the chin cup 2 of the crossing strap device 30 can be seen. The two straps 4', 4" are shown in a crossed position.

The first crossing strap 4' is attached to the lower part of the chin cup 2, the second crossing strap 4" is attached to the upper part of the chin cup 2. By arranging the straps 4', 4" in a crossover, the chin cup 2 is formed by the two straps 4', 4" without an additional part.

As can best be seen in Fig. 1C, the first crossing strap 4' is attached essentially in an angle of 45° upwards measured against the plane P so that a pull force on the first crossing strap 4' will apply a defined force to the portions V1, V2 lying above and in front of the ear opening in the cap device 50.

The second crossing strap 4" is attached essentially parallel to the plane P so that a pull force on the second crossing strip 4" will apply a force towards the portions V3, V4 lying behind the ear opening and in the neck region.

The pull can be applied to strap ends 14 that are secured with a ladder buckle 10 each.

As on the right hand side of the cap device 50, the forces applied to the textile material of the cap device 50 are fan like, the foci of the fans being the closing buckle 7 on the right hand side and the crossing straps 4', 4" on the left hand side respectively. This allows an even distribution of the forces around the cap device 50 and leads to a secure fit to the head H.

As mentioned above, it is the purpose of the cap device 50 that a plurality of sensors 70 can be securely and precisely placed on the head H of a person. Generally, the sensors are connected via cables with a processing device (not shown here). With 20, 40 or more sensors 70 applied to the head H, this results in many cables which have to be guided somehow, typically towards the neck of the person.

In one embodiment of a cap device 50, the neck portion CN is stiffened with a neck stiffening device 8, e.g., a neoprene lining or a different material somewhat stiffer than the textile material of the top portion CT or the side portions CL, CR. Neoprene is a good choice as it is also cushioning the neck against the cables running down from the cap device along the neck.

In Fig. 5, one embodiment of the neck stiffening device 8 is shown without the cap device 50, which also has some cable guiding means in form of a cable fastening element 1, here a Velcro fastener. The cables 12 run from the top of the cap device 50 towards the neck region where they are bundled and held by the Velcro fastener 1. For reasons of simplicity only two cables 12 are shown here.

The embodiment shown in Fig. 5 shows a further cable guiding means, i.e. a cable tree 11 in which cables 12 can be fitted in a form lock by inserting them in slots; here six slots are provided. The cable tree 11 allows a parallel arrangement of the cables 12 at the cap device 50.

In Fig. 6, a detail in the neck portion of an embodiment of a cap device 50 is shown. Underneath the textile, a neoprene neck stiffening device 8 as described in connection with Fig. 5 is positioned. As the neoprene material has certain stiffness and a certain thickness, holes can be drilled into it which can be used to hold the cable tree 11. Underneath the cable tree 11 a Velcro fastener 1 is located which can bundle and fix the cables coming from the sensors of the cap device 50.

Here, the Velcro fastener 1 and one cable tree 11 are used at the same time. It is also possible to use only the Velcro fastener 1 or the cable tree 11. If the number of cables is high, more than one cable tree 11 or a cable tree 11 with more than six slots can be used.

In other embodiments, the sides can be reversed, i.e. the two crossing straps 4', 4" of the crossing strap device 30 could attach to the right hand side of the head H, as the closing buckle 7 would attach to the left hand side of the head H. It is also possible that closing buckles 7 are used on both sides of the crossing strap device 30.

It is important to note, that the parts and the arrangement of the parts in the positioning means 2, 3, 4, 7, 9, 10, 14, 15, 30 can be different in other embodiments. For example, the closing buckle 7 or the ladder buckle 10 can be of a different design. The ladder buckle 10 as a stop for the strap can be e.g. a clamp device. It is also possible that the closing buckle 7 is replaced with a permanent fixture to the crossing straps 9. It is also possible that other embodiments use less parts for the positioning means.

Another means for fastening the cap device 50 to the head H is the face string 5 (see Fig. 2D) which is positioned in a fold running from the left side of the cap device 50, across the forehead part to the right hand side of the cap device 50. At one end of the face string 5, a loose end can be pulled to tighten the fit of the cap device 50 around the face of the person. A cord stopper 6 (see e.g. Fig. 2F for an embodiment with a clamp mechanism) keeps the tight fit as it blocks the movement of the face string 5.

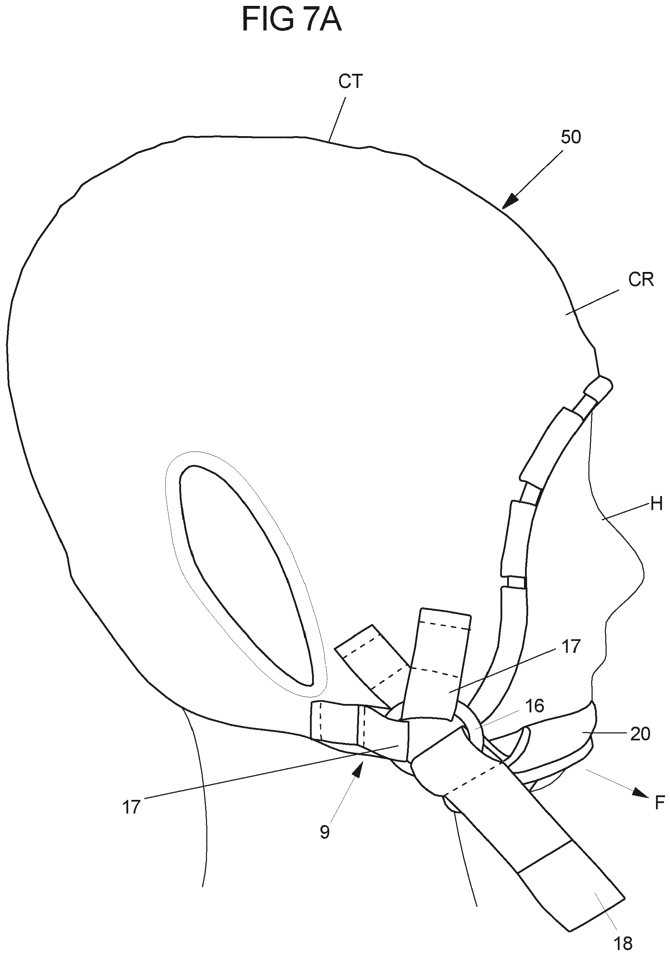

In Fig. 7A to 7C a further embodiment is shown, using a different arrangement for the positioning means, but otherwise conforming to the design described in connection with Fig. 1 to 6 above. Hence, the above description is generally applicable, so that reference is made here chiefly to the alternative arrangement of the positioning means.

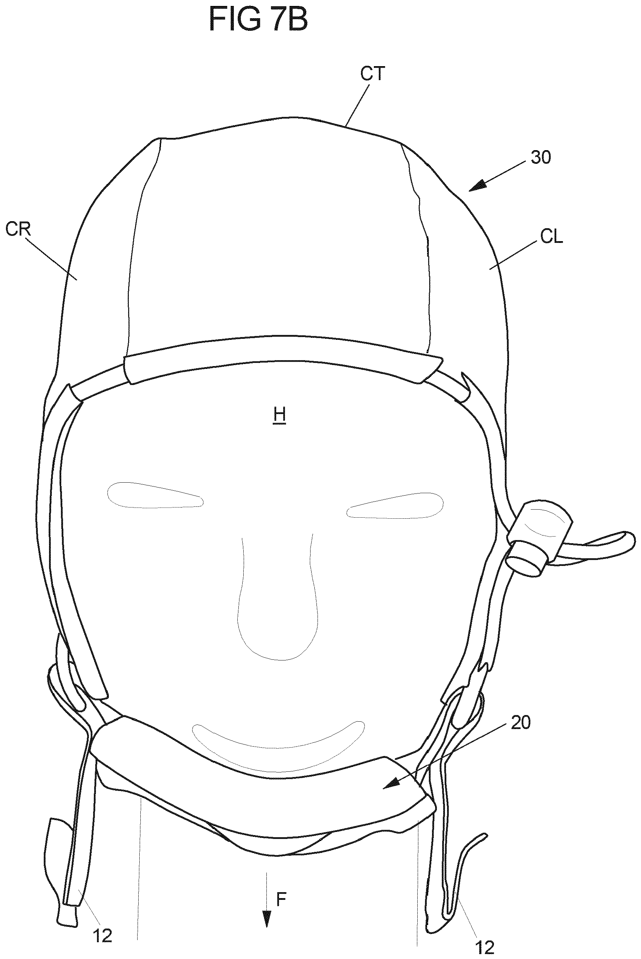

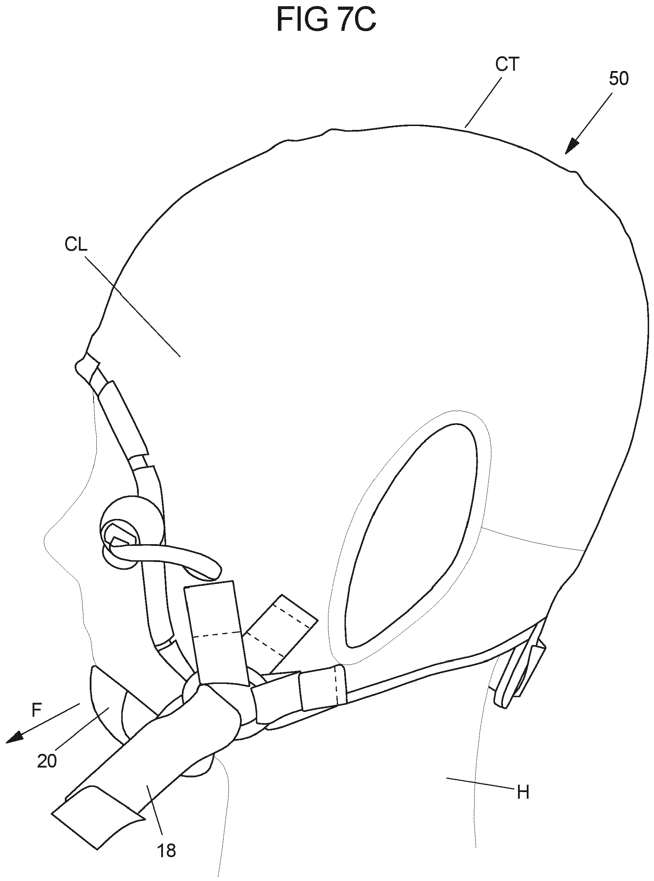

Fig. 7A shows a view of the right-hand side of the head H, Fig. 7B shows a frontal view of the head H and Fig. 7C shows a left-hand side of the head H.

The cap device 50 is mounted on a model of a head H. The chin belt device 20 itself is functionally equivalent to the one e.g. shown in connection with Fig. 3 but the attachments to left side CL and the right-side CR of the cap device 50 is different.

In the embodiment shown in Fig. 7A the positioning means on the right-hand side CR comprises a ring device 16 forming a connection point for different straps forming two loops 17. The ring device 16 is a closed shape which does not necessarily have a circular form. In the embodiment shown e.g in Fig. 2A buckle was used as an anchor point. The straps forming the two loops 17 are functionally similar to the crossing straps 9 discussed above.

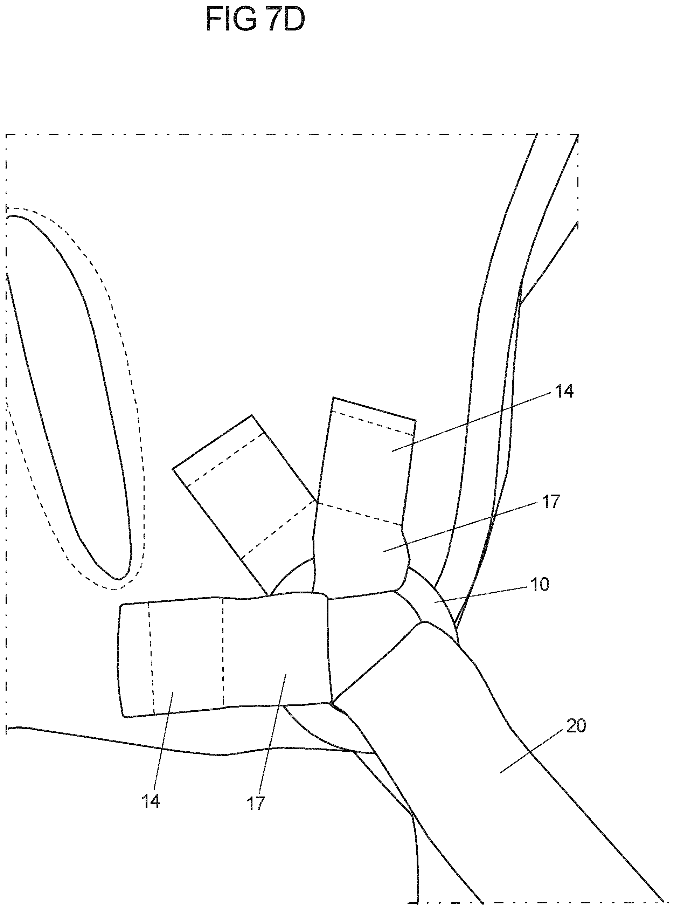

The ring device 16 is attached to the two loops 17 formed by the straps attached to the right-hand CR of the cap device 50. Details of the ring device 16 and the loops 17 can be seen in Fig. 7D.

By forming the loops 17, crossing straps are formed (best seen in Fig. 7D) i.e., the strap ends 14 fan out on the side of the cap device 50 to allow a defined and spread application of a pull force applied to the cap device 50.

Essentially the same design for an anchor point for the chin belt device 20 is used on the left-hand side CR of the cap device 50, as best seen in Fig. 7C.

The chin device 20 as can be seen in Fig. 7B, has on its right-hand side CR and left-hand CL side a long strap 17, each of which is lead through one of the ring devices 16.

The long straps 18 can be pulled with both hands in a forward direction. Due to the arrangement of the strap devices 14 as the positioning means a force vector F is generated in the median plane M (not shown here) of the person's body and at an angle α between 10 and 45° measured against the plane P (not shown here) perpendicular to the median plane M, approximately crossing the neck of the person in a standing position. The head dummy in Fig. 7A to 7C show a head approximately in a standing position.

In Fig. 8 to 14 the positioning of sensors 70 such as optodes in specific pattern is described.

In Fig. 8A, 8B a head H is shown in a medial sectional view (Fig. 8A) and a top view (Fig. 8B) showing defining positions which are used for finding a positional pattern for connection means 60 which will be described below.

In the lateral view of Fig. 8A the line from the nasion to the inion is taken as a length of 100%. This is divided into three sections (10%, 20%, 20%) from the nasion to the vertex and in three sections (20%, 20%, 10%) from the vertex to the inion.

In the top view of Fig. 8B the line from the nasion to the ear is divided into three sections (10%, 20%, 20%). The line from the ear to the inion is also divided into three sections (20%, 20%, 10%).

With these two lines a kind of coordinate system can be defined on the calotte of the head H as it is indicated by dashed lines in Fig. 8A, 8B. The intersections of the lines can define certain positions on the head H which are marked by circles. In other embodiments a finer separation (and thus positional pattern) by using 10 and 5 (and less) percent instead of the 20% section for creating the positions.

The vertex can be used to define a pattern for connections means 50 for a plurality of sensors 70 where all or at least some of the connection means 60 (see Fig. 14) are located in a pattern comprising regular triangles 61 or regular hexagons 62. The location of the connection means 60 for sensors 70 is important as the location of the sensors 70 needs to be known for making accurate measurements and / or for applying correct neurostimuli. In particular, the location of the sensors 70 relative to each other needs to be known, as well as the absolute location relative to the head H.

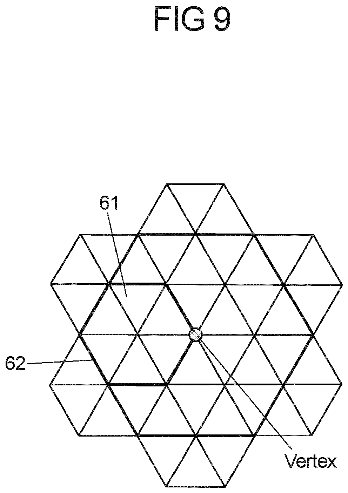

As shown in Fig. 9, a hexagonal pattern can be centered around the vertex. A regular hexagon 62 is indicated by bold lines. The side length of the hexagon in this example is 16 mm. A can be seen in Fig. 9, the regular hexagon 62 comprises six regular (equilateral) triangles 61, each having a side length of 16 mm.

Based on this pattern, larger regular hexagons with a side length of 32 mm can be defined. This larger regular hexagon 62 comprises 24 regular triangles with a side length of 16 mm.

In any case the base pattern can be centered around the vertex to provide a base for the connection means.

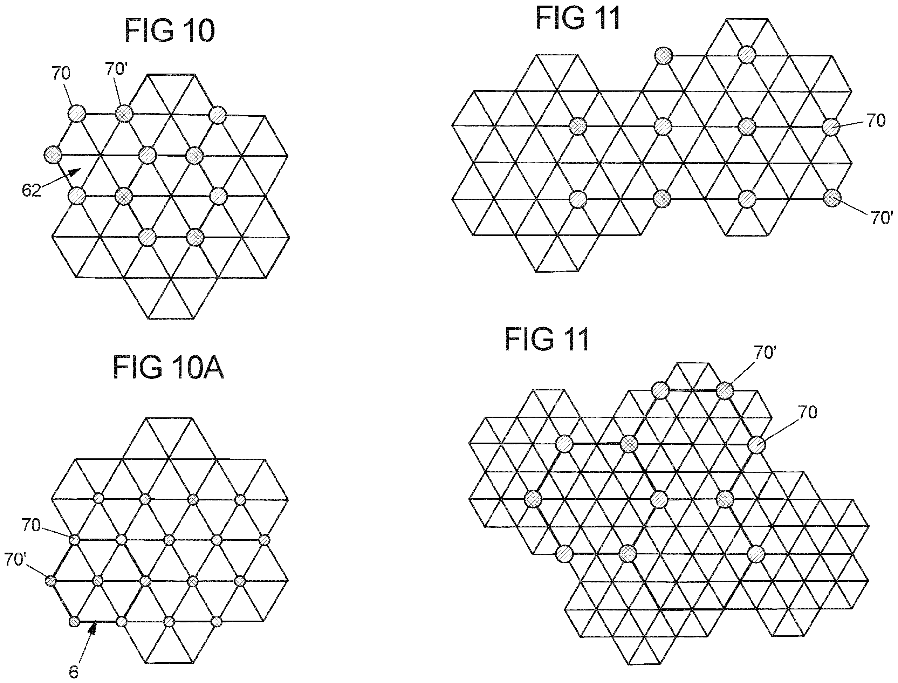

In general, regular polygons (e.g. triangles, hexagons) have sides of equal lengths and equal internal angles. As will be shown in connection with Fig. 11, more general hexagonal patterns can be used.

In Fig. 10, 11 is shown that the base pattern with a hexagon side length of 16 mm, and the extended pattern with a hexagon side length of 32 mm can be used for different sensor 70 arrangements. Two different types of sensors 70 are indicated by different hatching.

In Fig. 10 the pattern with the 16 mm hexagonal side length is used of a high-density sensor 70 arrangement. The embodiment assumes that two different kind of sensors 70, 70' are used, three of each kind in each regular hexagonal pattern. The two different kind of sensors 70, 70' are alternately placed on the circumference of the hexagon 62. The minimal distance between two sensors 70, 70' is 16 mm. No sensor is placed in the center of the hexagon 62.

In Fig. 10A a variation of the pattern - also based on a hexagonal side length of 16 mm - is shown. Here seven sensors 70, 70' are placed in hexagonal pattern, six sensors 70, 70'on the vertices, one sensor 70, 70' in the center.

In Fig. 11 a rectangular pattern is superimposed on a hexagonal pattern, which is not a regular hexagonal pattern, as the side lengths vary. A vertical side here is 32 mm long, the angled side is 42,52 mm long.

In Fig. 11A sensors are arranged as the embodiment of Fig. 10, but on a regular hexagon 62 with a side length of 32 mm.

The sizing of the hexagons 62 and triangles 61 is understood as exemplary, as other dimensions or units could be used.

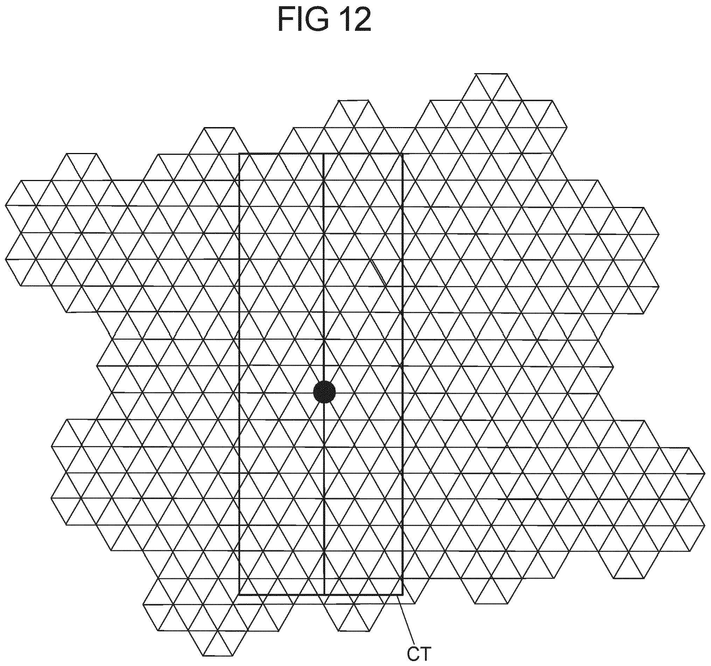

In Fig. 12 a hexagonal pattern is shown. Superimposed on the pattern, a rectangle representing the cap device top portion CT is shown. The rectangle is centered around the vertex represented by the dark circle. The dark circle is an "anchor point for co-registration of a grid pattern and an anatomical landmark of the head used for instance Cz in the EEG 10-20 system and subsystems.

The long axis of the rectangle comprises a little more than eight regular hexagons. The small axis is little less than three hexagons wide. It should be emphasized that the number of hexagons depends on the size of the cap device 50 and / or the density of the sensors 70 so that the arrangement in Fig. 12 is just an example. When manufacturing an embodiment of the cap device 50 using this design, the cap device top portion CT would be placed so that the vertex is placed on the vertex as defined in connection with Fig. 8A, 8B.

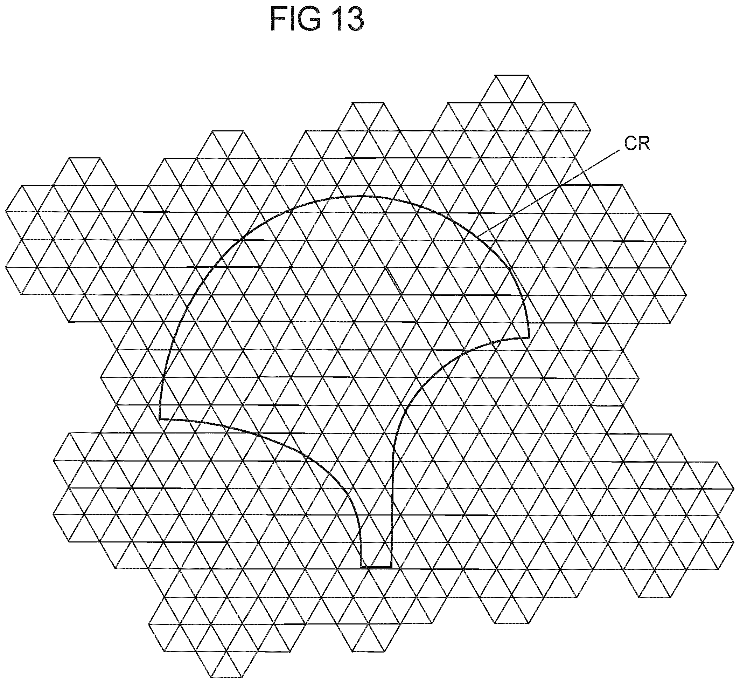

Fig. 13 then shows exemplary the cap device right CR superimposed on a hexagonal pattern.

Together with a cap device left side portion CL (not shown here), the right-side CR and the top portion CT can be sewed together forming the main part of the cap device 50. This ensures, that the same hexagon pattern is distributed over the cap device 50 allowing clearly defined sensor and / or stimulator positions based on the hexagon patterns. The relative positions remain unchanged if the cap device 50 material is evenly stretched.

The embodiments of Fig. 12 and 13 are only exemplary. For instance, the shown hexagonal pattern does not have to be regular/continuous across the whole panel CR/CL portions. It also should be noted that any displayed 2D projection cannot not properly represent the 3D pattern on the head H.

In Fig. 14 a hexagonal pattern with one regular hexagon highlighted by a thick line is shown. For reasons of visibility not all positions on the hexagons (i.e. vertices, centers) are occupied by connections means 60 for sensors 70 or sensors 70. In the highlighted hexagon, three connection means 60 for sensors are shown without sensors; two connection means on two vertices, one in the center. On one vertex a sensor 70 connected to a connection means 60 is shown.

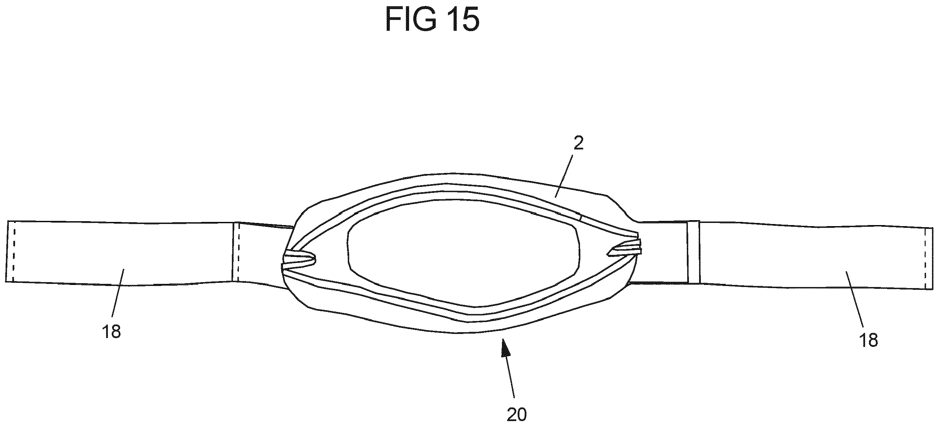

Fig. 15 shows a chin device 20 which can e.g. be used in connection with the embodiment shown in Fig. 7B.

As previously mentioned, the pattern described herein to place sensors 70 can also be used to place stimulators.

### Reference numbers

- 1: cable fastening element, Velcro fastener of the cable management device
- 2: chin cup
- 3: strap (right hand side)
- 4: crossing straps of crossing strap device (adjustable, left-hand side)
- 4': first crossing strap, left hand side
- 4": second crossing strap, left hand side
- 5: face string
- 6: cord stopper
- 7: closing buckle
- 7': closing buckle, female part
- 7": closing buckle, male part
- 8: neck stiffening device, neoprene lining
- 9: crossing straps attached to the cap device (right hand side)
- 10: ladder buckle

- 11: cable guide element, cable tree
- 12: cable

- 14: strap ends
- 15: short strap forming a loop
- 16: ring device
- 17: loops formed by straps
- 18: long straps

- 20: chin device

- 30: crossing strap device

- 50: cap device
- 60: connection means for a sensor, e.g. an optode
- 61: (regular) triangular pattern for connection means for sensors
- 62: (regular) hexagonal pattern for connection means for sensors

- 70: sensor

- α: angle of force vector against the plane perpendicular to the median plane
- CL: cap device left side
- CN: cap device neck portion
- CR: cap device right side
- CT: cap device top portion

- F: force vector
- H: head of a person
- M: median plane of the person
- P: plane perpendicular to median plane
- S1: first stretch direction
- S2: second stretch direction

- V1: frontal pull region
- V2: median pull region
- V3: median pull region
- V4: lower pull region

## Claims

1. Cap device (50) for use in taking of measurement data, in particular electrical or optical neuroimaging data from a head (H) of a person and / or for use in stimulating the neural functions of a person, with the cap device (50) comprising
a chin belt device (20) comprising positioning means (2, 3, 4, 7, 9, 10, 14, 15, 30) for defining chin belt device's (20) position relative to the head (H) and / or the face of the person wearing the cap device (50),
the positioning means (2, 3, 4, 7, 9, 10, 14, 15, 30) configured to define - when fastened - a force vector (F) in a median plane (M) of the person's body and at an angle (α) between 10 and 45° measured against a plane (P) perpendicular to the median plane (M), approximately crossing the neck of the person in a standing position.

2. Cap device (50) for use in taking of measurement data, in particular electrical or optical neuroimaging data from a head (H) of a person and / or for use in stimulating the neural functions of a person,, with the cap device (50), in particular according to claim 1, comprising connections means (60) for a plurality of sensors (70), in particular optodes, and / or stimulators wherein, at least a part of the connection means (60) is located in a pattern comprising triangles (61) or hexagons (62), in particular regular triangles (61) or regular hexagons (62).

3. Cap device (50) according to claim 1 or 2, wherein the chin belt device (20) with the positioning means (2, 3, 4, 7, 9, 10, 14, 15, 30) is fastenable on the chin or under the chin of the person.

4. Cap device (50) according to at least one of the preceding claims, wherein the chin belt device (20) is removable from the cap device (50).

5. Cap device (50) according to at least one of the preceding claims, wherein the positioning means (2, 3, 4, 7, 9, 10, 14, 15) comprises a crossing strap device (30), in which a
first crossing strap (4') is attachable to the cap device (50) towards the neck portion of the cap device (50), the first crossing strap being in use essentially parallel to the plane (P) perpendicular to the neck of the person in a standing position, and an
upper strap (4") attachable to the side portion of the cap device (50), the crossing straps (4', 4") crossing over in attaching to the chin belt device (20) to tighten the fit on the face of the person.

6. Cap device (50) according to at least one of the preceding claims, with a face string (5) for fastening the cap device (50) around the face of the person, in particular wherein the face string (5) is operable with one hand.

7. Cap device (50) according to claim 5 or 6, wherein the face string (5) is fixed to a part of the cap device (50).

8. Cap device (50) according to at least one of the preceding claims, with at least one cable guiding means (1, 11) for cables (12) attachable to sensors and / or stimulators at and / or on the cap device (50) positioned on the neck portion (CN) of the cap device (50).

9. Cap device (50) according to claim 8, comprising at least one Velcro fastener (1) for fastening cables (12) at the neck portion (CN) of the cap device (50).

10. Cap device (50) according to claim 8 or 9, comprising at least one cable guide element, in particular at least one cable tree (11) at the neck portion (CN) of the cap device (50).

11. Cap device (50) according to at least one of the preceding claims, having an elastic textile material at and / or in the top and the side portions (CT, CL, CR) of the cap device (50) with an even stretch capability at least in two perpendicular directions.

12. Cap device (50) according to at least one of the preceding claims, with a neck stiffening device (8), in particular comprising a neoprene lining, at and / or in the neck portion (CN) and / or the lower part of the side portion (CL, CR) of the cap device (50).

13. Cap device (50) according to claim 12, wherein at least a part of the cable guiding means (1, 11) is connectable to the neck stiffening device (8).

14. Cap device (50) according to at least one of the preceding claims, wherein all parts are manufactured from plastic, textiles and / or non-magnetic material.
